# EUROPÄISCHE PATENTANMELDUNG

(11) **EP 1 302 467 A2**
(43) Veröffentlichungstag der Anmeldung: **16.04.2003**
(21) Anmeldenummer: 02021002.7
(22) Anmeldetag: 20.09.2002
(51) Int. Cl.: C07D 277/12

(54) **Verfahren zur Herstellung von substituierten Thiazolinen und deren Zwischenprodukte**

(30) Priorität: 16.10.2001 AT 16392001
(71) Anmelder: DSM Fine Chemicals Austria Nfg GmbH & Co KG, 4021 Linz (AT)
(72) Erfinder: Krich, Sylvia, Dr., 4203 Altenberg b. Linz (AT); Rieder, Alexander, Dr., 6250 Kundl (AT); Heu, Ferdinand, 4030 Linz (AT); Steinbauer, Gerhard, Dr., 4470 Enns (AT)
(74) Vertreter: Lindinger, Ingrid

(57) **Zusammenfassung**

Verfahren zur Herstellung von substituierten Thiazolinen der Formel (1) 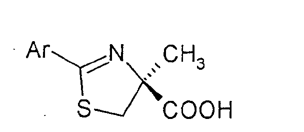 in der Ar einen Phenyl-, Naphthyl-, Thienyl-, Pyridyl- oder Chinolinylrest bedeuten, der gegebenenfalls durch ein oder mehrere Substituenten aus der Gruppe Halogen, OH, Benzyloxy, C₁-C₄-Alkyl, C₁-C₄-Alkoxy, COOR₁ mit R₁ gleich H oder C₁-C₄-Alkyl substituiert sein kann,
durch Kopplung von (S)-α-Methylcystein-Hydrochlorid der Formel (II) 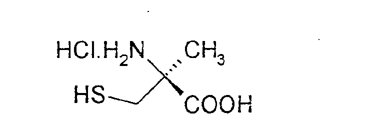 mit einem Nitril der Formel (III)

Ar-CN

in der Ar wie oben definiert ist, oder einem korrespondierenden C₁-C₄-Alkylimidat, bei welchem (S)-α-Methylcystein-Hydrochlorid der Formel (II) in einem geeigneten Lösungsmittel mit an einem Nitril der Formel (III) oder einem korrespondierenden C₁-C₄-Alkylimidat in Gegenwart einer tertiären Base bei einem pH-Wert von 6.5 bis 10 bei 50°C bis zur Rückflusstemperatur zu dem entsprechenden Thiazolin der Formel (I) umgesetzt wird, sowie Verfahren zur Herstellung von (S)-α-Methylcystein-Hydrochlorid und dessen Verwendung zur Herstellung von Thiazolinen der Formel (I).

## Beschreibung

Substituierte Thiazoline, wie etwa 4,5-Dihydro-2-(2,4-dihydroxyphenyl)-4-methylthiazol-4-(S)-carbonsäure, 4,5-Dihydro-2-(2,3-dihydroxyphenyl)-4-methyl-thiazol-4-(S)-carbonsäure, 4,5-Dihydro-2-(2-hydroxy-3-methoxyphenyl)-4-methylthiazol-4-(S)-carbonsäure, 4,5-Dihydro-2-(4-carboxy-2-hydroxyphenyl)-4-methylthiazol-4-(S)-carbonsäure, 4,5-Dihydro-2-(2-hydroxyphenyl)-4-methylthiazol-4-(S)-carbonsäure, 4,5-Dihydro-2-(3-hydroxyquinolin-2-yl)-4-methylthiazol-4-(S)-carbonsäure, 4,5-Dihydro-2-(2-hydroxynaphth-1-yl)-4-methylthiazol-4-(S)-carbonsäure, 4, 5-Dihydro-2-(3-hydro-xynaphth-2-yl)-4-methylthiazol-4-(S)-carbonsäure, 4,5-Dihydro-2-(2-hydroxy-phenyl-methyl)-4-methylthiazol-4-(S)-carbonsäure, u.s.w. sind potentielle Eisenchelatoren: Die Herstellung dieser Verbindung erfolgt über die Kopplung des (S)-α-Methylcysteins oder eines Salzes davon mit der entsprechenden Nitril- oder Imidoesterverbindung und wurde bereits in der Literatur beschrieben. So erfolgt die Kopplung von (S)-α-Methylcystein-Hydrochlorid mit 2,4-Dihydroxy-benzonitril beispielsweise gemäß J. Med. Chem. 1999, 42,2432-2440 in entgastem Methanol durch 3-tägiges Erhitzen auf 71°C unter Zusatz von entgastem Phosphatpuffer, zur Erzielung eines pH-Wertes von 6, wodurch nach Aufarbeitung des Reaktionsgemisches 4,5-Dihydro-2-(2,4-dihydroxyphenyl)-4-methylthiazol-4-(S)-carbonsäu-re in 57%iger Ausbeute erhalten wird. Analog wird 4,5-Dihydro-2-(2-hydroxyphenyl)-4-methylthiazol-4-(S)-carbonsäure in einer Ausbeute von lediglich 32% hergestellt. Eine wichtige Zwischenverbindung für die Synthese der oben angeführten Zielverbindungen ist dabei das (S)-α-Methylcystein, das beispielsweise gemäß Tetrahedron 1993, 49 (24), 5359-5364 in einer Seebach-analogen Synthese durch saure Hydrolyse von 2S,4S-Methyl-2-tert.-butyl-1,3-thiazolidin-3-formyl-4-methyl-4-carboxylat erhalten wird. 2S,4S-Methyl-2-tert.-butyl-1,3-thiazolidin-3-formyl-4-methyl-4-carboxylat wird dabei ausgehend von (S)-Cysteinmethylester und Pivaldehyd über 2S-Methyl-2-tert.-butyl-1,3-thiazolidin-4-carboxylat, Einführung einer Formylschutzgruppe zu 2S,4S-Methyl-2-tert.-butyl-1,3-thiazolidin-3-formyl-4-carboxylat, Reaktion bei -78°C mit Lithiumdiisopropylamid zum korrespondierenden Enolat und Quenchen des Enolates mit Methyliodid hergestellt. Die Ausbeute an (S)-α-Methylcystein ausgehend von (S)-Cysteinethylester beträgt dabei lediglich 29%. Neben der geringen Ausbeute an (S)-α-Methylcystein sind die aufwendigen Verfahrensschritte und vor allem das Ausgangsprodukt (S)-Cysteinmethylester-Hydrochlorid, einer unnatürlichen, kommerziell nicht verfügbaren und daher für technische Synthesen nicht in Betracht zu ziehende Verbindung, wesentliche Nachteile dieser Herstellvariante.

In Synlett (1994), Seite 702-703 werden (R)-und (S)-Methylcysteinethyiester-Hydrochlorid durch Reaktion von Ethylbenzimidat-Hydrochlorid mit L-Cysteinethylester-Hydrochlorid, anschließender Methylierung und Racemattrennung des dadurch erhaltenen racemischen Gemisches von 4-Carbethoxy-4-methyl-2-phenylthiazolin mittels präparativer HPLC auf Cellulosetriacetat, sowie saurer Hydrolyse der erhaltenen enantiomerenreinen (R) und (S)- 4-Carbethoxy-4-methyl-2-phenyl-thiazoline mit nachfolgender Veresterung hergestellt. (R)-Methyl-Cysteinethylester-Hydrochlorid wird sodann mit Cyanomethyl-diphenylphosphinoxid zum korrespondierenden Thiazolin kondensiert. In weiterer Folge wird gemäß Synlett Thiangazol als Endprodukt hergestellt.

Aufgabe der Erfindung war es ein im technischem Maßstab durchführbares Verfahren zu finden, das die Herstellung von substituierten Thiazolinen in im Vergleich zum Stand der Technik höheren Ausbeuten bei einfacherer Reaktionsführung gewährleistet.

Gegenstand der Erfindung ist daher ein Verfahren zur Herstellung von substituierten Thiazolinen der Formel (I) 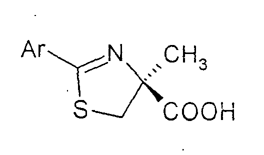 in der Ar einen Phenyl-, Naphthyl-, Thienyl-, Pyridyl- oder Chinolinylrest bedeuten, der gegebenenfalls durch ein oder mehrere Substituenten aus der Gruppe Halogen, OH, Benzyloxy, C₁-C₄-Alkyl, C₁-C₄-Alkoxy, COOR₁ mit R₁ gleich H oder C₁-C₄-Alkyl substituiert sein kann,
durch Kopplung von (S)-α-Methylcystein-Hydrochlorid der Formel (II) 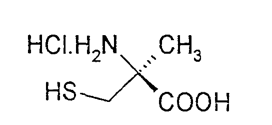 mit einem aromatischen oder heteroaromatischen Nitril der Formel (III)

Ar-CN

in der Ar wie oben definiert ist oder einem korrespondierenden C₁-C₄-Alkylimidat, das dadurch gekennzeichnet ist, dass (S)-α-Methylcystein-Hydrochlorid der Formel (II) in einem C₁-C₄-Alkohol, einem Gemisch aus zwei oder mehreren C₁-C₄-Alkoholen oder einem Gemisch aus C₁-C₄-Alkohol und einem Kohlenwasserstoff mit 0,5 bis 2 mol an einem Nitril der Formel (III) oder einem korrespondierenden C₁-C₄-Alkylimidat pro mol (S)-α-Methylcystein-Hydrochlorid in Gegenwart von 1,5 bis 3 mol einer tertiären Base pro mol (S)-α-Methylcystein-Hydrochlorid bei einem pH-Wert von 6.5 bis 10 bei 50°C bis zur Rückflusstemperatur zu dem entsprechenden Thiazolin der Formel (I) umgesetzt wird, das durch Entfernen des Lösungsmittels, Extraktion der Verunreinigungen und Ausfällen des Thiazolins durch Ansäuern aus dem Reaktionsgemisch isoliert wird.

Durch das erfindungsgemäße Verfahren werden substituierte Thiazoline der Formel (I) hergestellt.
In der Formel (I) bedeutet Ar einen Phenyl-, Naphthyl-, Thienyl-, Pyridyl- oder Chinolinylrest, wobei der Phenyl- und Naphthylrest bevorzugt sind. Besonders bevorzugt bedeutet Ar Phenyl.

Die Reste können dabei gegebenenfalls auch ein oder mehrfach durch Substituenten aus der Gruppe Halogen, wie Fluor, Chlor, lod oder Brom, OH, Benzyloxy, C₁-C₄-Alkyl, wie Methyl, Ethyl, Propyl und Butyl, C₁-C₄-Alkoxy,wie Methoxy, Ethoxy, Butoxy, COOR₁ mit R₁ gleich H oder C₁-C₄-Alkyl substituiert sein. Bevorzugte Substituenten sind Cl, OH, Methyl, Methoxy und COOH, besonders bevorzugt ist OH.
Bevorzugt ist Ar durch einen oder zwei, besonders bevorzugt durch zwei, der oben angeführten Substituenten substituiert.
Mögliche bevorzugte Endverbindungen, die erfindungsgemäß hergestellt werden können, sind beispielsweise 4,5-Dihydro-2-(2,4-dihydroxyphenyl)-4-methylthiazol-4-(S)-carbonsäure, 4,5-Dihydro-2-(2,3-dihydroxyphenyl)-4-methylthia-zol-4-(S)-carbonsäure, 4,5-Di-hydro-2-(2-hydroxy-3-methoxyphenyl)-4-methylthiazol-4-(S)-carbonsäure, 4,5-Dihy-dro-2-(4-carboxy-2-hydroxyphenyl)-4-methylthiazol-4-(S)-carbonsäure, 4,5-Dihydro-2-(2-hydroxyphenyl)-4-methylthiazol-4-(S)-carbonsäure, 4,5-Di-hydro-2-(3-hydroxy-quinolin-2-yl)-4-methylthiazol-4-(S)-carbonsäure, 4,5-Dihydro-2-(2-hydroxynaphth-1-yl)-4-methylthiazol-4-(S)-carbonsäure, 4,5-Dihydro-2-(3-hydroxy-naphth-2-yl)-4-methylthiazol-4-(S)-carbonsäure, 4,5-Dihydro-2-(2-hydroxy-phenyl-methyl)-4-methyl-thiazol-4-(S)-carbonsäure, u.s.w.

Als Ausgangsverbindung werden (S)-α-Methylcystein-Hydrochlorid der Formel (II) und ein Nitril der Formel (III) oder ein korrespondierendes Imidat eingesetzt.

(S)-α-Methylcystein-Hydrochlorid kann beispielsweise gemäß dem Stand der Technik, wie etwa gemäß Synlett (1994), Seite 702-703 hergestellt werde. Bevorzugt wird (S)-α-Methylcystein-Hydrochlorid jedoch nach einer modifizierten Variante hergestellt, die ebenfalls Gegenstand der Erfindung ist.
Bei dieser modifizierten Variante werden in der ersten Stufe L-Cystein-Verbindungen der Formel (IV) 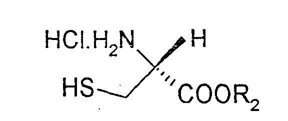 in der R₂ H oder C₁-C₄-Alkyl bedeutet, mit gegebenenfalls substituierten Nitrilen der Formel (V)

Ar-CN

in der Ar einen Phenyl-, Naphthyl-, Thienyl-, Pyridyl- oder Chinolinylrest bedeutet, der gegebenenfalls durch ein oder mehrere Substituenten aus der Gruppe Halogen, OH, Benzyloxy, C₁-C₄-Alkyl, C₁-C₄-Alkoxy, COOR₁ mit R₁ gleich H oder C₁-C₄-Alkyl substituiert sein kann, wobei die OH-Gruppen gegebenenfalls durch eine geeignete Schutzgruppe, wie etwa Tetrahydropyranyl, Acetyl, Benzyl usw. geschützt sein können, oder einem korrespondierendem C₁-C₄-Alkylimidat, in Anwesenheit einer tert. Base, wie Triethylamin, Di-i-Propyl-Ethylamin, Pyridin u.s.w. in einem C₁-C₄-Alkohol, wie Methanol, Ethanol, Butanol, einem Gemisch aus zwei oder mehreren C₁-C₄-Alkoholen oder einem Gemisch aus C₁-C₄-Alkohol und einem Kohlenwasserstoff, wie etwa Toluol, oder einem Alkan, z.B. Hexan, Heptan u.s.w., zu einer Verbindung der Formel (VI) 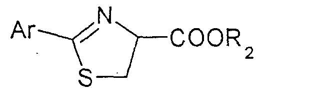 in der Ar und R₂ wie oben definiert sind,
gekoppelt.
Als L-Cysteinverbindungen der Formel (IV) eignen sich L-Cystein, sowie die L-Cystein-C₁-C₄-ester-Hydrochloride.
Die L-Cysteinverbindungen der Formel (IV) wird sodann entweder mit einem entsprechende Nitril der Formel (V) oder dem korrespondierendem C₁-C₄-Alkylimidat HCl umgesetzt.

Als Nitrile eignen sich dabei Benzonitril, Tolunitril oder an den Sauerstoffatomen beispielsweise mit Tetrahydropyranyl, Acetyl oder Benzyl geschütztes 2,4-Dihydroxybenzonitril.
Die korrespondierenden C₁-C₄-Alkylimidate sind entweder käuflich erwerbbar oder können aus den Nitrilen mit einem C₁-C₄-Alkohol und HCI hergestellt werden.
Das Nitril der Formel (V) bzw. das entsprechende Alkylimidat wird dabei bevorzugt in einer äquimolaren Menge bezogen auf die L-Cysteinverbindung der Formel (IV) eingesetzt. Gegebenenfalls kann auch ein Überschuss eingesetzt werden, wodurch jedoch keine Vorteile erzielt werden. Die Kopplung mit der L-Cysteinverbindung erfolgt in Anwesenheit einer tert. Base, wie Triethylamin, Di-i-Propyl-Ethylamin oder Pyridin.
Bevorzugt wird Triethylamin verwendet.
Die tert. Base wird in einer Menge von 0,9 bis 1,3 mol, bevorzugt von 1,0 bis 1,2 mol und besonders bevorzugt von 1,02 bis 1,1 mol pro mol L-Cysteinverbindung zugesetzt. Größere Überschüsse können auch eingesetzt werden, wirken sich aber negativ auf die Ausbeute aus.
Verbindungen der Formel (VI) sind beispielsweise 4-Carbethoxy-2-phenylthiazolin, 2-Phenylthiazolin-4-carbonsäure, 4-Carbethoxy-2-(2,4-ditetrahydropyranyl)thiazolin usw..
Wird als L-Cysteinverbindung L-Cystein eingesetzt, so wird eine Verbindung der Formel (VI) mit R₂ gleich H erhalten, die sodann in einen C₁-C₄-Ester überführt wird. Die Verbindung der Formel (VI), in der R₂ C₁-C₄-Alkyl bedeutet, wird sodann in einer 2.Stufe, nach wässriger Aufarbeitung und Extraktion, gegebenenfalls azeotrope Trocknung der Extrakte und/oder gegebenenfalls Isolierung der Verbindung, mit einem Methylierungsreagens, beispielsweise mit Methyliodid, -chlorid oder -bromid, unter Einwirkung einer Base zum entsprechenden Thiazolin der Formel (VII) 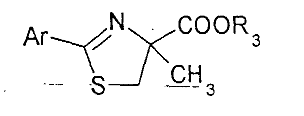 in der Ar wie oben definiert ist und R₃ C₁-C₄-Alkyl bedeutet, wie etwa 4-Carbethoxy-4-methyl-2-phenylthiazolin oder 4-Carbethoxy-4-methyl-2-(2,4-ditetrahydropyranyl) thiazolin usw. umgesetzt.

Dieser 2.Schritt erfolgt in einem Ether, beispielsweise in Methyl-tert.butylether (MTBE), Tetrahydrofuran (THF) oder Diethylether, und in Anwesenheit einer Base statt. Als Base wird bevorzugt Lithiumdiisopropylamid (LDA) verwendet, das aus Diisopropylamin und Butyllithium erhalten wird.
Das Methylierungsmittel wird dabei in einer Menge von 1 bis 3 mol, bevorzugt von 1 bis 2 mol und besonders bevorzugt von 1,2 bis 1,7 mol pro mol Verbindung der Formel (VI) eingesetzt.
LDA wird in einer Menge von 1 bis 1,5 mol, bevorzugt von 1 bis 1,3 mol und besonders bevorzugt von 1,05 bis 1,2 mol pro mol Verbindung der Formel (VI) eingesetzt.
Die Methylierung erfolgt bei einer Temperatur von -80°C bis +40°C, bevorzugt von -30°C bis +35°C und besonders bevorzugt von -20°C bis +30°C.
Nach erfolgter Umsetzung wird soviel HCI zugegeben, dass ein pH-Wert zwischen 1,5 und 5, bevorzugt zwischen 2 bis 3 erreicht wird, worauf ein zweiphasiges Gemisch erhalten wird.
Die organische Phase, die das Thiazolin der Formel (VII) enthält, kann direkt, nach Extraktion, in die nächste Stufe eingesetzt werden. Das Thiazolin der Formel (VII) kann jedoch auch zuerst isoliert werden.
Bei der 3. Stufe wird durch basische Hydrolyse, sowie gegebenenfalls nach Abspaltung einer Schutzgruppe an einer oder mehreren am aromatischen Rest befindlichen Hydroxygruppe(n) die korrespondierende Thiazolincarbonsäure der Formel (VIII) 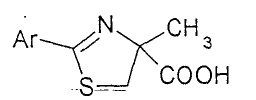 in der Ar wie oben definiert ist, wie etwa gegebenenfalls substituierte 4-Methyl-2-phenylthiazolin-4-carbonsäure usw. als Racemat erhalten. Dies erfolgt durch Zugabe einer Base, wie etwa NaOH, KOH, LiOH usw., oder einem Gemisch aus Base und THF oder Dioxan, bevorzugt zu der Produktlösung aus Stufe 2, wodurch ein zweiphasiges Reaktionsgemisch erhalten wird, das bei Rückflusstemperatur gerührt wird. Nach dem Abkühlen auf Raumtemperatur wird die wässrige Phase mit HCI versetzt, bis ein pH-Wert zwischen1,5 bis 4,5, bevorzugt zwischen 1,5 bis 4 und besonders bevorzugt von 1,5 bis 3 erreicht ist. Durch das Sauerstellen scheidet sich die Säure als Öl ab, das gegebenenfalls noch durch Extraktion, Abtrennung der organische Phase, Entfernen von Wasser durch azeotrope Destillation und Abdestillieren des Extraktionsmittels isoliert werden kann.
Als 4.Schritt erfolgt die Racemattrennung mit einer Spaltbase. Als Spaltbase eignen sich beispielsweise enantiomerenreine, chirale Amine, wie etwa gegebenenfalls substituiertes Phenylethylamin, gegebenenfalls substituiertes Phenylglycinamid, Norephedrin, Chinin u.s.w..
Die Spaltung erfolgt in einem Lösungsmittel, in dem sich die Thiazolin-4-carbonsäure der Formel (VIII) löst, beispielsweise in einem Alkohol, wie etwa Ethanol, Propanol u.s.w., einem Alkohol/Wasser-Gemisch, einem Essigsäureester, wie etwa Isopropylacetat (IPA), Essigsäureethylester, u.s.w.. Nach der Zugabe der Spaltbase wird unter Rühren mit der Kristallisation durch Abkühlen des Reaktionsgemisches begonnen. Dazu kann beispielsweise ein mehrstufiges Temperaturprogramm, bei welchem die Abkühlgeschwindigkeit in den einzelnen Stufen variiert, gewählt werden. Bei der Kristallisation ist es auch möglich diese durch Animpfen zu unterstützen bzw. auszulösen. Bevorzugt wird nach dem Animpfen eine Zeit lang etwas langsamer abgekühlt und gegen Ende der Kristallisation, zur vollständigen Ausfällung, wieder etwas schneller abgekühlt. Die Abkühlung erfolgt von Raumtemperatur auf bis zu -15°C. Nach beendeter Kristallisation wird filtriert, die Kristalle bevorzugt gewaschen und anschließend gegebenenfalls getrocknet.
Die so erhaltenen Salze der Carbonsäure werden zur Aufreinigung sodann bevorzugt umkristallisiert. Dazu werden die erhaltenen Kristalle bei erhöhter Temperatur in einem geeigneten Lösungsmittel gelöst und das gereinigte Carbonsäuresalz wiederum durch Abkühlen unter Rühren auskristallisiert. Auch hier ist ein mehrstufiges Temperaturprogramm möglich.
Nach abgeschlossener Kristallisation wird das entsprechende chirale Carbonsäuresalz wiederum abfiltriert und gegebenenfalls getrocknet.
Zur Freisetzung der entsprechenden chiralen Säure wird sodann zu einem Gemisch aus chiralem Salz, Wasser und einem geeigneten Lösungsmittel HCI zugegeben, wodurch zwei klare Phasen erhalten werden. Als Lösungsmittel eignen sich solche, die mit Wasser nicht mischbar sind, die die Säure lösen und die gegen HCI resistent sind. Beispiele dafür sind Ether oder Ester. Bevorzugt werden Ether eingesetzt, besonders bevorzugt MTBE. Die HCI-Zugabe erfolgt so lange, bis der pH-Wert der wässrigen Phase 1 bis 4, bevorzugt 1 bis 3 und besonders bevorzugt 1,5 bis 3 beträgt. Dann wird die organische Phase abgetrennt und gegebenenfalls gewaschen. Nach Abtrennung des Lösungsmittels wird die (S)-Thiazolincarbonsäure der Formel (VIII), beispielsweise (S)-4-Methyl-2-phenylthiazolin-4-carbonsäure oder (S)-4-Methyl-2-(2,4-dihydroxyphenyl)thiazolin-4-carbonsäure usw. erhalten.

Anstelle der Racematspaltung durch Spaltbasen kann jedoch auch eine lipasenkatalysierte Racemattrennung des Thiazolins der Formel (VII) durchgeführt werden.
Geeignete Lipasen sind beispielsweise Pig Liver Esterase, Candida rugosa Lipase, Aspergillus species Lipase, Aspergillus species Protease, Bacillus species Protease,
Subtilisin Carlsberg, Candida antarctica "A" Lipase, Candida antarctica "B" Lipase, ChiroCLEC_{TM-PC} (dry), ChiroCLEC_{TM-PC} (slurry), u.s.w..

Die (S)-Thiazolincarbonsäure der Formel (VIII) wird in weiterer Folge durch saure Hydrolyse in (S)-2-Methylcystein HCl überführt.
Dazu wird die (S)-Thiazolincarbonsäure der Formel (VIII) in HCI gelöst und die so erhaltene Lösung bei Rückflusstemperatur gekocht. Bevorzugt wird dabei unter Stickstoffatmosphäre gearbeitet. Nach 1 bis 30 Stunden, bevorzugt 5 bis 25 Stunden und besonders bevorzugt nach 10 bis 20 Stunden wird das Rektionsgemisch auf 0 bis 35°C, bevorzugt auf 10 bis 30°C und besonders bevorzugt auf 10 bis 20°C abgekühlt und das ausgefallene Spaltprodukt, beispielsweise gegebenenfalls substituierte Benzoesäure, abgetrennt, welches sodann mit gegebenenfalls entgastem Wasser oder mit verdünnter HCI ein- oder mehrmals gewaschen wird. Das saure Filtrat wird gegebenenfalls zuerst zur vollständigen Entfernung des Spaltproduktes mit einem geeigneten Extraktionsmittel extrahiert. Beispiele für geeignete Lösungsmittel sind MTBE, Toluol, Ethylmethylketon, IPA, THF, CH₂Cl₂, Diethylether u.s.w.. Die Isolierung von (S)-2-Methylcystein HCl kann sodann mittels Sprühtrocknung der wässrigen Phase erfolgen. !n einer anderen Variante wird die wässrige Phase, bevorzugt unter reduziertem Druck, eingedampft, wodurch ein Öl erhalten wird, das in weiterer Folge 1 bis 5mal in gegebenenfalls entgastem Wasser, oder einem Gemisch aus Wasser mit Tetrahydrofuran (THF), Aceton oder IPA gelöst wird und jeweils zur Gänze oder bis zu einer 65 bis 20%igen Lösung, bevorzugt unter reduziertem Druck, eingedampft wird.
Wird das Reaktionsgemisch nicht zur Gänze eingedampft, so wird der so erhaltenen konzentrierten Lösung ein für die azeotrope Destillation geeignetes Lösungsmittel, das mit Wasser ein Azeotrop bildet, beispielsweise Toluol, Dichlormethan, 2-Butanol, i-Propanol oder MTBE, zugesetzt und das in der Lösung enthaltene Wasser ausgeschleppt. Die verbleibende Suspension oder Lösung, die das (S)-2-Methylcystein HCl, enthält, kann sodann direkt in die nächste Stufe, die Kopplung mit einem Nitril der Formel (III), eingesetzt werden. Gegebenenfalls wird dazu die Lösung bzw. Suspension mit frischem Lösungsmittel ergänzt und anschließend direkt mit dem Nitril der Formel (III) in Gegenwart einer tertiären Base zum gewünschten Endprodukt, einem Thiazolin der Formel (I) umgesetzt.
Die verbleibende Suspension oder Lösung kann aber auch gegebenenfalls zum Ausfällen des (S)-2-Methylcystein HCl mit einem Ether, wie etwa MTBE, Diethylether, Diisopropylether u.s.w., oder einem Kohlenwasserstoff, wie etwa einem C₆-C₈-Alkan, Toluol, u.s.w., versetzt werden, worauf das (S)-2-Methylcystein HCl abfiltriert, ein oder mehrere Male mit dem verwendeten Ether oder Kohlenwasserstoff gewaschen und unter Vakuum getrocknet wird.

Durch diese erfindungsgemäße Methode wird (S)-2-Methylcystein HCl in kristalliner Form in einer Ausbeute von bis zu 95% erhalten.

Eine weitere erfindungsgemäße Herstellungsvariante für (S)-2-Methylcystein HCl ist eine modifizierte Variante der in Tetrahedron (1993), 49 (24), S.5359-5364 beschriebenen Seebach-analogen Synthese, die auf der durch eine chirale Schutzgruppe induzierten enantioselektiven Methylierung des unnatürlichen und daher kommerziell nicht verfügbaren S-Cysteinmethylester HCl beruht.
Bei der erfindungsgemäßen Variante wird das leicht verfügbare L-Cysteinethylester HCl in einer ersten Stufe mit 2 bis 3 Äquivalenten Aceton in einem Kohlenwasserstoff, beispielsweise Toluol, Hexan, u.s.w. oder in Aceton selbst, gegebenenfalls in Anwesenheit eines tert. Amines, wie Triethylamin, Di-i-Propyl-Ethylamin oder Pyridin u.s.w, umgesetzt. Nach dem Abtrennen des Reaktionswassers wird das Reaktionsgemisch abgekühlt, der Niederschlag abfiltriert, gegebenenfalls gewaschen und das Filtrat vom Lösungsmittel befreit. Wird Aceton selbst als Lösungsmittel eingesetzt, so entfällt das Abtrennen des Reaktionswassers. Die Reaktionslösung wird lediglich eingeengt, der Rückstand zwischen NaHCO₃, NaOH oder KOH und Toluol verteilt und das Produkt aus der organischen Phase isoliert.
Das so erhaltene Acetonid wird in der 2.Stufe in 2 bis 3 Äquivalenten Ameisensäure gelöst, mit Toluol oder Essigsäureanhydrid versetzt und das erhaltene Reaktionsgemisch für einige Zeit, bevorzugt für 10 min bis zu 5 Stunden gerührt. Danach wird das Reaktionsgemisch eingeengt und zwischen einem Ether, Ester oder geeigneten Kohlenwasserstoff und einer Natriumhydrogencarbonat-, NaOH- oder KOH-Lösung verteilt. Die organische Phase wird azeotrop getrocknet und vom Lösungsmittel befreit.
In der 3.Stufe erfolgt die Methylierung des aus der 2.Stufe erhaltenen N-formylierten Acetonids (3-Formyl-2,2-dimethyl-thiazolin-4-carbonsäure-ethylester) durch Deprotonierung mit 1 bis 1,5 Äquivalenten, bevorzugt von 1,1 bis 1,2 Äquivalenten an LDA und anschließender Umsetzung mit 1 bis 2 Äquivalenten, bevorzugt mit 1,1 bis 1,5Äquivalenten Methylierungsmittel.

Als Lösungsmittel eignen sich beispielsweise MTBE, THF, Diethylether, u.s.w.. Gegebenenfalls kann auch ein Co-Solvent, wie etwa 1,3-Dimethyl-3,4,5,6-tetrahydro-(1H)-pyrimidinon (DMPU) zugesetzt werden. Die Reaktionstemperatur liegt bei -25 bis -80°C, bevorzugt bei -35 bis -75°C und besonders bevorzugt bei -45 bis -55°C.
Anschließend erfolgt eine wässrige Aufarbeitung.
Die Enantiomerentrennung des erhaltenen racemischen 3-Formyl-2,2,4-trimethylthiazolin-4-carbonsäure-ethylester erfolgt sodann bevorzugt mittels selektiver Esterhydrolyse unter Verwendung einer Lipase, beispielsweise mit Pig Liver Esterase, Candida rugosa Lipase, Aspergillus species Lipase; Aspergillus species Protease, Bacillus species Protease, Subtilisin Carlsberg, Candida antarctica "A" Lipase, Candida antarctica "B" Lipase, ChiroCLEC_{TM-PC} (dry), ChiroCLEC_{TM-PC} (slurry), u.s.w..

Nach erfolgter Enantiomerentrennung erfolgt in der 5.Stufe die Hydrolyse zu (S)-2-Methylcystein HCl analog dem vorher beschriebenen Verfahren, wobei die Abtrennung der Benzoesäure entfällt.

Das gemäß dem Stand der Technik oder bevorzugt das gemäß einer der erfindungsgemäßen Variante, bevorzugt nach der modifizierten Synlett-Variante hergestellte (S)-2-Methylcystein HCl wird sodann mit einem aromatischen oder heteroaromatischen Nitril der Formel (III) Ar-CN oder einem korrespondierendem C₁-C₄-Alkylimidat gekoppelt.
In der Formel (III) hat Ar dieselbe Bedeutung wie in Formel (I).
Die Nitrile sind zum Teil käuflich erwerbbar, können aber auch aus den entsprechenden Aldehyden, beispielsweise mittels Ameisensäure, Natriumformiat und Hydroxylammoniumsulfat, hergestellt werden.
Die korrespondierenden C₁-C₄-Alkylimidate können aus den Nitrilen mit einem C₁-C₄-Alkohol und HCI hergestellt werden.

Das entsprechende Nitril oder das Imidat wird sodann erfindungsgemäß mit (S)-2-Methylcystein HCl gekoppelt.

Die Kopplung erfolgt in einem C₁-C₄-Alkohol, einem Gemisch aus zwei oder mehreren C₁-C₄-Alkoholen oder einem Gemisch aus C₁-C₄-Alkohol und einem Kohlenwasserstoff mit 0,5 bis 2 mol an einem Nitril der Formel (III) bzw an dem korrespondierenden C₁-C₄-Alkylimidat pro mol (S)-α-Methylcystein-Hydrochlorid in Gegenwart von 1,5 bis 3 mol einer tertiären Base pro mol (S)-α-Methylcystein-Hydrochlorid. Bevorzugt werden 0,8 bis 1,5 mol, besonders bevorzugt 0,9 bis 1,1 mol Nitril bzw. Imidat eingesetzt. Als tert. Base eignen sich beispielsweise Triethylamin, Di-i-Propyl-Ethylamin oder Pyridin, die bevorzugt in einer Menge von 1,8 bis 2,5 mol, besonders bevorzugt 1,9 bis 2,2 mol Amin pro mol (S)-α-Methylcystein-Hydrochlorid zugesetzt werden. Als Lösungsmittel eignen sich C₁-C₄-Alkohole, wie Methanol, Ethanol Butanol, ein Gemisch aus zwei oder mehreren C₁-C₄-Alkoholen oder ein Gemisch aus C₁-C₄-Alkohol und einem Kohlenwasserstoff, wie etwa Toluol, C₆-C₈-Alkan, u.s.w.. Bevorzugt wird jedoch Ethanol und /oder Butanol eingesetzt.
Durch die Zugabe der tert. Base wird in der Reaktionslösung ein pH-Wert von 6.5 bis 10, bevorzugt von 7 bis 9 und besonders bevorzugt von 7.5 bis 8 eingestellt.
Das Reaktionsgemisch wird unter Rühren für 1 bis 40 Stunden, bevorzugt für 5 bis 30 Stunden und besonders bevorzugt für 15 bis 25 Stunden, bevorzugt unter Stickstoffatmosphäre, auf 50°C bis zur Rückflusstemperatur erhitzt.
Danach wird gegebenenfalls abgekühlt und das Lösungsmittel, bevorzugt unter reduziertem Druck, abdestilliert, wodurch ein Öl erhalten wird.
Aus diesem Öl wird das entsprechenden Thiazolin der Formel (I) sodann durch Extraktion der Verunreinigungen und Ausfällen des Thiazolins durch Ansäuern aus dem Reaktionsgemisch isoliert.
Dazu wird das Öl zwischen Wasser und einem geeigneten Lösungs- bzw. Extraktionsmittel verteilt und bei einem geeigneten pH-Wert überschüssiges Edukt extrahiert. Beispielsweise wird bei Verwendung von 2,4-Dihydroxynitril das Öl in Wasser aufgenommen, der pH-Wert mit einer geeigneten Base, beispielsweise mit KOH, NaOH, u.s.w. auf einen pH-Wert von Wert von 6.5 bis 9, bevorzugt von 7 bis 9 und besonders bevorzugt von 7.5 bis 8 eingestellt und ein oder mehrere Male mit einem geeigneten Extraktionsmittel, wie etwa Diethylether, MTBE, Dichlormethan, Essigsäureethylester, u.s.w. extrahiert.
Die wässrige Phase wird abgetrennt und mit einer geeigneten Base, beispielsweise mit KOH, NaOH, u.s.w. auf einen pH-Wert von Wert von 10 bis 14, bevorzugt von 11 bis 13 eingestellt. Danach erfolgt erneut eine ein- oder mehrmalige Extraktion mit einem geeigneten Extraktionsmittel, wie etwa Diethylether, MTBE, Dichlormethan, Essigsäureethylester, u.s.w.. Anschließend wird gegebenenfalls filtriert, beispielsweise mit Aktivkohle und sodann mit einer geeigneten Säure, wie etwa HCI, H₂SO₄, HBr u.s.w. ein pH-Wert von 6.5 bis 10, bevorzugt von 7 bis 9 und besonders bevorzugt von 7.5 bis 8 eingestellt. Aus der Wasserphase wird sodann das Extraktionsmittel vollständig durch Destillation, bevorzugt unter reduziertem Druck, entfernt.
Die Extraktions- bzw. Reinigungsschritte bei unterschiedlichem pH-Wert können auch in unterschiedlicher Reihenfolge durchgeführt werden.
Die verbleibende wässrige Lösung wird mit einer geeigneten Säure, wie etwa HCI H₂SO₄, HBr u.s.w. solange angesäuert bis ein pH-Wert von 1 bis 3, bevorzugt von 1 bis 2 erreicht wird. Dabei beginnt das gewünschte Thiazolin auszukristallisieren. Die so erhaltene Suspension wird sodann noch für einige Zeit, bevorzugt für 10min bis 3 Stunden, besonders bevorzugt für 30min bis 1,5 Stunden bei einer Temperatur von 5 bis 25°C, bevorzugt von 10 bis 20°C gerührt, anschließend der Feststoff abfiltriert, gewaschen und unter Vakuum getrocknet.

Durch das erfindungsgemäße Kopplungsverfahren werden Thiazoline der Formel (I) im Vergleich zum Stand der Technik wesentlich höheren Ausbeuten von bis zu 99% erhalten.

### Beispiel 1: Herstellung von (S)-2-Methylcystein HCl

### 1. Stufe: Kopplung zum 4-Carbethoxy-2-phenylthiazolin

100 g (0.54 mol, 1.00 eq) Ethylbenzimidat·HCl und 100 g (0.54 mol, 1.00 eq) Cysteinethylester·HCl wurden in einem 500 ml Schmizo, ausgestattet mit einem Rückflusskühler, in 500 ml Ethanol suspendiert. Das Ethanol wurde zuvor entgast, indem man es zweimal unter Stickstoffatmosphäre bis zum Rückfluss erhitzte. Nach Zugabe von 57.4 g (0.567 mol, 1.05 eq) Triethylamin wurde das Reaktionsgemisch 2 h rückflußgekocht. Das Ethanol wurde unter reduziertem Druck (100 mbar) abdestilliert (455 ml Destillat), wodurch man eine ölige gelbe Suspension erhielt. Zu dieser wurden 450 ml MTBE gegeben. Dieses Gemisch wurde mit 200 ml 0.5 M HCl und zweimal mit 200 ml Wasser gewaschen. Die organische Phase wurde abgetrennt und das restliche Wasser durch eine azeotrope Destillation (Wasserabscheider, 4 h, 10 ml Wasser) ausgekreist. Dann wurden noch 125 ml MTBE abdestilliert, um den Wassergehalt so weit wie möglich zu reduzieren. Es wurden 400 ml einer hellgelben MTBE-Lösung (Wasserwert nach Karl Fischer: < 0.05 %w/w), erhalten, welche das Produkt 4-Carbethoxy-2-phenylthiazolin enthält. Diese Lösung wurde in Stufe 2 eingesetzt

### 2. Stufe: Methylierung zum 4-Carbethoxy-4-methyl-2-phenylthiazolin

152.2 g (0.594 mol, 1.10 eq) Butyllithiumlösung (25 %ig in Heptan) wurden unter Stickstoff innerhalb von 30 min zu einer Lösung von 90.8 ml (65.6 g, 0.648 mol, 1.20 eq) Diisopropylamin in 800 ml absolutem MTBE bei -15 °C zugetropft. Diese Lösung wurde 30 min bei -15 °C gerührt. 400 ml der MTBE-Lösung mit dem 4-Carbethoxy-2-phenylthiazolin wurden innerhalb von 30 min bei -15 °C zugetropft und 1 h bei -15 °C gerührt, wodurch eine braune Lösung entstand. 115.0 g (50.4 ml, 0.81 mol, 1.50 eq) Methyliodid wurden zugegeben und das Reaktionsgemisch auf 23 °C erwärmt und weitere 3 h bei dieser Temperatur gerührt. Anschließend wurden 300 ml 2 M HCI langsam unter starker Rührung zugegeben, dann auf pH 2-3 gestellt, sodass die Temperatur nicht über 30 °C stieg. Das dadurch erhaltene zweiphasige Gemisch wurde noch 30 min lang gerührt. Dann wurde die organische Phase abgetrennt. Diese MTBE-Lösung, enthaltend 4-Carbethoxy-4-methyl-2-phenylthiazolin, wurde in Stufe 3 eingesetzt.

### 3. Stufe: Basische Hydrolyse zur rac. 4-Methyl-2-phenylthiazolin-4-carbonsäure

Zu der Produktlösung aus Stufe 3 (1340 m!) wurden 270 ml 4 M NaOH (2 eq) zugegeben. Dieses zweiphasige Gemisch wurde unter Rührung 1 h (52 °C Siedetemperatur) rückflussgekocht. Nach Abkühlen auf Raumtemperatur wurden die Phasen getrennt. Nach Abdestillieren von Ethanol von der wässrigen Phase unter reduziertem Druck (50 mbar, 40 °C, 100 ml Destillat) wurde diese wieder mit 250 ml MTBE gewaschen. Die wässrige Phase wurde abgetrennt und mit 6 M HCI auf pH 2-3 gestellt, wobei die Temperatur nicht 35 °C überstieg. Beim Sauerstellen scheidet sich rac. 4-Methyl-2-phenylthiazolin-4-carbonsäure als ein Öl ab. Um eine schöne Phasentrennung zu erreichen, wurden 100 ml MTBE zugegeben, gut geschüttelt und die organische Phase abgetrennt. Die wässrige Phase wurde mit 200 ml MTBE gewaschen. Die vereinigten MTBE-Phasen wurden mit 100 ml Wasser gewaschen. Dann wurde von der MTBE-Lösung, die die Säure enthielt, das Wasser mittels azeotroper Destillation entfernt. Das MTBE wurde unter reduziertem Druck bei 50 °C abdestilliert, wodurch ein braunes Öl erhalten wurde. Dieses wurde noch einmal in 200 ml MTBE aufgenommen und MTBE wieder abgezogen bis man die racemische 4-Methyl-2-phenylthiazolin-4-carbonsäure als Öl erhalten hat.
Gesamtausbeute Stufe 1-3: 92,9 g racemische 4-Methyl-2-phenylthiazolin-4-carbonsäure (77,7% d.Th.)

### 4. Stufe: Racemattrennung mit (R)-Phenylethylamin über ein diastereomeres Salz

### Kristallisation:

92.9 g (0.42 mol, 1.00 eq) der racemischen 4-Methyl-2-phenylthiazolin-4-carbonsäure wurden in 370 ml Isopropylacetat in einem 500 ml Schmizo gelöst. Zu dieser Suspension wurden 40.1 ml (38.2 g, 0.75 eq) (R)-Phenylethylamin gegeben, wobei sich sofort eine klare Lösung bildete, mit welcher die Kristallisation unter Rührung gemäß nachfolgendem Temperaturprogramm durchgeführt wurde:
a.) in 30 min von Raumtemperatur auf 15 °C;
b.) in 60 min von 15 auf 10 °C;
c.) in 10 h von 10 auf -10 °C, bei -10 °C über Nacht rühren.

Bei 10 °C wurde die Lösung mit Kristallen des Phenylethylamin (PE)-Salzes angeimpft. Die Kristallisation setzte hierauf bei 7 °C ein. Nach abgeschlossener Kristallisation wurde filtriert und die Kristalle zweimal mit je 50 ml kaltem Isopropylacetat gewaschen. Das gelbe PE-Salz wurde bei 50 °C unter Vakuum getrocknet.
Ausbeute: 48.0 g (33.4 % d. Th., ee% = 91.5 (GC))

### Umkristallisation:

48.0 g (0.14 mol) des PE-Salzes wurden in 450 ml Isopropylacetat in einem 500 ml Schmizo bei 90 °C Manteltemperatur gelöst. Es wurde eine klare, gelbliche Lösung erhalten, mit welcher unter Rührung die Umkristallisation gemäß nachfolgendem Temperaturprogramm durchgeführt wurde:
a.) in 2.5 von 90 auf 60 °C;
b.) in 4 h von 60 auf 20 °C;
c.) in 10 h von 20 auf -10 °C, bei -10 °C über Nacht rühren.

Bei 80 °C wurde die Lösung mit Kristallen des PE-Salzes angeimpft. Die Kristallisation setzte hierauf bei 75 °C ein. Nach abgeschlossener Kristallisation wurde filtriert und die Kristalle zweimal mit je 50 ml kaltem Isopropylacetat gewaschen (Chirale Reinheit > 99:1). Das gelbe PE-Salz wurde bei 50 °C unter Vakuum getrocknet
Ausbeute: 42.0 g (87.5 % d. Th., ee% = 98.7 (GC))

### Freisetzen der chiralen 4-Methyl-2-phenylthiazolin-4-carbonsäure:

Zu einem kräftig gerührten Gemisch von 42.0 g (0.12 mol) chiralem PE-Salz, 52.5 ml Wasser und 315 ml MTBE wurden langsam ca. 125 ml 1 M HCI zugegeben wobei zwei klare Phasen erhalten wurden. Die HCl-Zugabe erfolgte solange, bis der pH-Wert der wässrigen Phase 2-3 betrug. Die organische Phase wurde abgetrennt und zweimal mit je 105 ml Wasser gewaschen. Von der organischen Phase wurde MTBE abgezogen, wodurch die chirale 4-Methyl-2-phenylthiazolin-4-carbonsäure als gelbes Öl erhalten wurde, welches über Nacht bei Raumtemperatur durchkristallisierte.
Ausbeute: 26.0 g (95.8 % d. Th.)

### Alternative zu Stufe 3 und 4:

Als Alternative zu den Stufen 3 und 4 wurde eine Racemattrennung von 4-Carbethoxy-4,5-dihydro-4-methyl-2-phenylthiazolin mittels Lipase durchgeführt: Herstellung einer Stammlösung: 300 mg 4-Carbethoxy-4,5-dihydro-4-methyl-2-phenylthiazolin wurden in 26ml Phosphatpuffer aufgenommen und durch Zugabe von 1 ml DMF und 1ml Isopropylacetat gelöst. Von dieser Stammlösung wurden 2ml zu ca. 20 mg Candida rugosa Lipase zugegeben und 24 Stunden bei Raumtemperatur gerührt.
Nach 4 Stunden wurde bei einer Ausbeute von 79% ein ee von 70%, nach 24 Stunden ein ee von 99% (bei 70% Ausbeute) mittels chiraler GC festgestellt.

Unter anderem konnten noch mit Candida antarctica A, Aspergillus species Protease und diversen CLECs ähnliche Ergebnisse erzielt werden.

### 5. Stufe: Saure Hydrolyse zum (S)-2-Methylcysteinhydrochlorid (Variante a)

In 65 ml 6 M HCI (entgast durch zweimaliges Aufkochen in Stickstoffatmosphäre) wurden 6.20 g (28.0 mmol) 4-Methyl-2-phenylthiazolin-4-carbonsäure gelöst. Diese Lösung wurde dann 16 h in Stickstoffatmosphäre rückflussgekocht. Nach Abkühlen auf Raumtemperatur wurde das Reaktionsgemisch filtriert, um die ausgefallene Benzoesäure (2.94 g = 85.3 %) abzutrennen, welche zweimal mit je 10 ml entgastem Wasser gewaschen wurde. Das HCI-saure Filtrat wurde anschließend dreimal mit je 30 ml MTBE extrahiert. Die wässrige Phase wurde unter reduziertem Druck zu einem Öl eingedampft, welches zweimal hintereinander in je 55 ml entgastem Wasser gelöst und immer bis zu einer ca. 50 %igen Lösung unter reduziertem Druck eingedampft wurde. Zu dieser konzentrierten Lösung wurden anschließend 150 ml MTBE (entgast durch zweimaliges Aufkochen in Stickstoffatmospäre) gegeben. Das Wasser wurde durch azeotrope Destillation (Wasserabscheider) bei 52 °C ausgeschleppt, wobei sich zunächst im Kolben ein Öl bildete, welches bei weiterem Azeotropisieren in einen Niederschlag von (S)-2-Methylcysteinhydrochlorid zerfiel. Nach Abdestillieren von ca. 40 ml MTBE und Abkühlen der Suspension auf 20 °C wurde das (S)-2-Methylcysteinhydrochlorid abfiltriert, zweimal mit je 20 ml MTBE gewaschen und unter Vakuum (20 mbar) bei 50 °C getrocknet.

### Ausbeute an (S)-2-Methylcysteinhydrochlorid : 4.10 g (85.0 % d. Th.);

5. Stufe: Saure Hydrolyse zum (S)-2-Methylcysteinhydrochlorid (Variante b) 18,6 g 4-Methyl-2-phenylthiazolin-4-carbonsäure wurden unter Stickstoffatmosphäre in 186 ml 6n HCI gelöst und dann für 16 Stunden unter Rückfluß erhitzt. Nach Abkühlen auf 20°C wurde die ausgefallene Benzoesäure abfiltriert und mit 2 x 10ml VE-Wasser nachgewaschen. Das Filtrat wurde dreimal mit je 95ml MTBE extrahiert, danach wurde restliches MTBE durch Evakuieren bei Raumtemperatur abgezogen und erst danach das Wasser bei vermindertem Druck und max. 40°C abdestilliert. Das erhaltene Öl wurde in 110ml Wasser gelöst und nochmals bis auf eine ca. 50%ige Lösung eingeengt. Zu dieser konzentrierten Lösung wurden 300ml 2-Butanol zugegeben und das restliche Wasser durch Abdestillieren des Azeotrops von 2-Butanol und Wasser (gesamt: 250 ml) ausgeschleppt. Dabei wurde ein Wassergehalt von 0,6gg% in der letzten Destillatfraktion erreicht. Die erhaltene konzentrierte Lösung wurde unter vermindertem Druck weiter eingeengt, auf Raumtemperatur gekühlt und danach mit 200ml MTBE versetzt. Die ausgefallenen Kristalle wurden abgesaugt, 2 x mit je 20ml MTBE nachgewaschen und bei 45°C / 30mbar getrocknet
Ausbeute: 13,62 g (94,5% d.Th.) Reinheit: 91,8gg% (quant. NMR)

### Beispiel 2: Herstellung von (S)-2-Methylcystein HCl (Variante 2)

### 1. Stufe - Acetonid-Synthese

50,0g (0,27 Mol) L-Cysteinethylester Hydrochlorid wurden in 500ml n-Hexan suspendiert und bei Raumtemperatur mit 31,4g (0,54 Mol) Aceton und 27,3g (0,27 Mol) Triethylamin versetzt. Danach wurde das Reaktionsgemisch am Wasserabscheider unter Abtrennung des Reaktionswassers für 2 Stunden zum Sieden erhitzt. Das Reaktionsgemisch wurde abgekühlt, der Niederschlag (Triethylamin Hydrochlorid) abfiltriert, mit Hexan gewaschen und das Filtrat vom Lösungsmittel befreit.

Ausbeute an 2,2-Dimethyl-thiazolin-4-carbonsäureethylester (Acetonid):
48,6 g (95% d.Th.) farbloses Öl; Reinheit: 99,8 Fl% (GC)

### 2. Stufe - N-Formylierung

45 g (0,24 Mol) 2,2-Dimethyl-thiazolin-4-carbonsäureethylester (Acetonid) wurden in 450 g Ameisensäure gelöst, mit 180 g Essigsäureanhydrid versetzt (exotherme Reaktion) und für eine Stunde gerührt. Das Reaktionsgemisch wurde am Rotavapor eingeengt und zwischen MTBE und Natriumhydrogencarbonat-Lösung verteilt. Die organische Phase wurde azeotrop gertocknet und dann vom Lösungsmittel befreit.

Ausbeute an 3-Formyl-2,2-dimethyl-thiazolin-4-carbonsäure-ethylester:
51g (98% d.Th.) farbloses Öl Reinheit: 98,2 Fl% (GC)

### 3. Stufe - Methylierung

12,1g (120mmol) Diisopropylamin wurden unter Stickstoffatmosphäre in 200ml MTBE gelöst, zu dieser Lösung wurden bei -20°C 28,1g (110mmol) einer 25%igen Butyllithium-Lösung in Heptan zugetropft und für 20 Minuten gerührt. Zu der so hergestellten LDA-Lösung wurden bei -50°C 21,7g (100mmol) 3-Formyl-2,2-dimethyl-thiazolin-4-carbonsäureethylester in 100ml MTBE innerhalb einer halben Stunde zugetropft. Dabei bildete sich eine orange Suspension. Nach einer Stunde Reaktionszeit wurden 7,4 ml (120 mmol) Methyljodid in 40 ml DMPU zugetropft und für weitere 1,5 Stunden bei -50°C gerührt und zuletzt auf Raumtemperatur erwärmt. Das Reaktionsgemisch wurde mit 125ml 1n HCI versetzt und die Phasen getrennt. Die wässrige Phase (pH 2,5) wurde mit 100ml MTBE extrahiert und die vereinten organischen Phasen zwei mal mit je 100ml gesättigter Natriumchlorid-Lösung gewaschen. Die organische Phase wurde über Natriumsulfat getrocknet, filtriert und das Lösungsmittel abgezogen (21,5 g).
Der Rückstand wurde bei 140-145°C / 4mbar destilliert und das Destillat (9,37g), der racemische 3-Formyl-2,2,3-trimethyl-thiazolin-4-carbonsäureethylester säulenchromatographisch über Kieselgel 60 mit Ethylacetat:Hexan = 1:5 gereinigt.

### 4. Stufe - Enantiomerentrennung

161, 4 mg Substrat (3-Formyl-2,2,3-trimethyl-thiazolin-4-carbonsäureethylester) wurden in 12ml Phosphatpuffer aufgenommen und mit 100mg DMF versetzt. Nach 7 Minuten Rühren wurde eine klare Lösung erhalten. Von dieser Stammlösung wurden 1,35ml (entspricht 18,83mg Substrat) zur Candida antarctica "B" Lipase zugesetzt und das Reaktionsgemisch 24h bei Raumtemperatur gerührt. Anschließend wurde das Produkt (3-Formyl-2,2,3-trimethyl-thiazolin-4-carbonsäure) durch Extraktion mittels MTBE mit einem ee von 80% isoliert.

### 5. Stufe - Hydrolyse zu (S)-2-Methylcystein HCl

140mg (0,6mmol) 3-Formyl-2,2,4-trimethyl-thiazolin-4-carbonsäureethylester wurden in 5ml 5n HCI für 20 Stunden zu Sieden erhitzt, zur Trockene eingedampft und anschließend zwei mal wieder in 5ml Wasser aufgenommen und neuerlich eingedampft.
Ausbeute: 100mg leicht gelbe Kristalle von (S)-2-Methylcystein HCl (~quantitativ)

### Beispiel 3: Herstellung von 2,4-Dihydroxybenzonitril

In einen 500 ml Schmizo wurden 50.0 g (0.362, 1.0 eq) 2,4-Dihydroxybenzaldehyd und 180 ml Ameisensäure gegeben, wodurch eine braune Suspension zustande kam. 45.8 g (0.673 mol, 1.8 eq) Natriumformiat wurden innerhalb von 2 min zuchargiert, wobei die Temperatur auf 33 °C anstieg. Nachdem das Reaktionsgemisch wieder auf 30 °C abgekühlt war, wurden 35.6 g (0.217 mol, 1.2 eq) Hydroxylammoniumsulfat innerhalb von 3 min zugegeben, woraus eine dicke braune Suspension resultierte. Nach 10 min Rühren bei 30 - 32 °C wurde daraus eine braune Lösung. Während des anschließenden Erwärmens auf 100 °C setzte bei 38 °C eine Kristallisation ein. Bei 70 °C ging der Kristallbrei in eine dünne Suspension über, welche wieder rührfähig war. Dieses Reaktionsgemisch wurde 2 h bei 100 °C gerührt, die Farbe wechselte dabei auf dunkelbraun. Ein IPC (Memi-IP-4_01) und ein DC (Kieselgel 60 F₂₅₄, Aceton:n-Hexan:Wasser 20:20:1) zeigte fast vollständigen Umsatz. Die Ameisensäure wurde unter reduziertem Druck abdestilliert (60 °C, 10 mbar, 170 ml Destillat). Der feste dunkelbraune Rückstand wurde in 400 ml MTBE bei 40 °C 1 h lang eingerührt. Der unlösliche Rückstand (62.5 g) wurde abfiltriert und zweimal mit je 50 ml MTBE gewaschen. Zur Mutterlauge gab man 10 g Aktivkohle (Norit CA 5) und das Gemisch wurde 1 h rückflussgekocht, bei 40 °C über Celite Super Hyflow filtriert und zweimal mit je 50 ml MTBE gewaschen. Die Mutterlauge wurde dreimal mit je 100 ml Wasser gewaschen. Nach dem azeotropen Auskreisen des Wassers aus der MTBE-Lösung wurde auf ca. 20 % des ursprünglichen Volumens unter reduziertem Druck eingeengt und 500 ml Toluol zugegeben. Das restliche MTBE wurde dann weiter abgezogen. Dabei fiel ein brauner Niederschlag aus, welcher abfiltriert wurde. Die Toluollösung wurde dann auf ein Volumen von ca. 150 ml eingeengt, wobei das 2,4-Dihydroxybenzonitril auskristallisierte. Dieses wurde abfiltriert, zweimal mit je 30 ml Toluol gewaschen und unter Vakuum getrocknet (45 °C, 20 mbar)
Ausbeute an 2,4-Dihydroxybenzonitril: 34.5 g (70.5 %, Reinheit 97 % (HPLC))

### Beispiel 4: Kopplung zu 4,5-Dihydro-2-(2,4-dihydroxyphenyl)-4-methylthiazol-4-(S)-carbonsäure

In 40 ml Ethanol (entgast durch zweimaliges Aufkochen in Stickstoffatmosphäre) wurden 4.00 g (23.3 mmol, 1.0 eq) (S)-2-Methylcysteinhydrochlorid, hergestellt nach Beispiel 1, und 3.14 g (23.3 mmol, 1.0 eq) 2,4-Dihydroxybenzonitril, hergestellt nach Beispiel 3, suspendiert und 4.95 g (6.8 ml, 48.9 mmol, 2.05 eq) Triethylamin zugegeben. Die erhaltene Suspension weist einen pH-Wert von 7.5 - 8.0 auf und wurde für 20 h unter Rührung und Stickstoff auf Rückflußtemperatur erhitzt. Dann wurde auf 20 °C abgekühlt und Ethanol unter reduziertem Druck abdestilliert wodurch ein Öl erhalten wurde. Dieses wurde in 50 ml Wasser gelöst, der pH-Wert mit ca. 1.0 ml 20 %i-ger KOH auf pH 7.5 eingestellt und dreimal mit j 20 ml MTBE extrahiert. Die wässrige Phase wurde abgetrennt und mit ca. 10.5 ml 20 %iger KOH auf pH 12 gestellt. Es wurde viermal mit je 20 ml MTBE extrahiert, bis ein Triethylamingehalt < 0.1 %area (IPC (GC)) erreicht wurde und dann mit ca 3.4 ml 6 M HCl auf pH 7.5 gestellt. Aus der Wasserphase wurde das MTBE vollständig mit einer Destillation unter reduziertem Druck entfernt (50 °C, 200 - 45 mbar, 8 ml Destillat). Die wässrige Lösung wurde mit ca. 4.6 ml 6 M HCl weiter auf pH 1.5 angesäuert. Dabei beginnt 4,5-Dihydro-2-(2,4-dihydroxyphenyl)-4-methylthiazol-4-(S)-carbonsäure auszukristallisieren. Diese Suspension wurde 1 h bei 15 °C gerührt. Der Feststoff wurde abfiltriert, zweimal mit je 10 ml Wasser (15 °C) gewaschen und unter Vakuum (30 mbar) bei 55 °C getrocknet.

### Ausbeute an 4,5-Dihydro-2-(2,4-dihydroxyphenyl)-4-methylthiazol-4-(S)-carbonsäure:

5.17 g (87.6 % d. Th., ¹H-NMR zeigte keine signifikanten Verunreinigungen)

### Beispiel 5: Kopplung zu 4,5-Dihydro-2-(2,4-dihydroxyphenyl)-4-methylthiazol-4-(S)-carbonsäure

5,80 g einer ~80%igen Lösung von (R)-2-Methylcysteinhydrochlorid in 2-Butanol (entspricht 5,38g bzw. 25mmol, 1,0 eq Reinsubstanz) (hergestellt nach Beispiel 1, 5. Stufe, Variante b, Produktlösung vor Fällung mit MTBE) und 3,41g (25mmol, 1,0 eq) 2,4-Dihydroxybenzonitril, hergestellt nach Beispiel 3, wurden in 55 ml 2-Butanol suspendiert und 5,18g (7,1 ml, 51,2 mmol, 2.05 eq) Triethylamin zugegeben. Die erhaltene Suspension wies einen pH-Wert von 7.5 auf und wurde für 20 h unter Rührung und Stickstoffatmosphäre auf Rückflußtemperatur erhitzt. Nach 2 Stunden war der pH-Wert auf 7,0 gefallen, daher wurden weitere 0,7 g (0,96 ml, 7 mmol, 0,25 eq.) Triethylamin zugegeben, worauf sich ein pH-Wert von 7,5 einstellte, der dann stabil blieb. Nach 20 Stunden Reaktionszeit wurde das Lösungsmittel unter reduziertem Druck abdestilliert, wodurch ein Öl erhalten wurde. Dieses wurde in 50 ml Wasser gelöst, der pH-Wert mit 20,5 ml 20 %iger KOH auf pH 12,5 eingestellt und dreimal mit je 30 ml MTBE extrahiert. Die wässrige Phase wurde abgetrennt und mit 6,0 ml 6 M HCI auf pH 7 gestelit, dann wurde das MTBE unter reduziertem Druck abgezogen (25-30 °C, bis 45 mbar). Die wässrige Lösung wurde mit 5,2 ml 6 M HCi weiter auf pH 2 angesäuert, dabei begann 4,5-Dihydro-2-(2,4-dihydroxyphenyl)-4-methylthiazol-4-(S)-carbonsäure auszukristallisieren. Diese Suspension wurde 1 h bei 20°C gerührt. Der Feststoff wurde abfiltriert, zweimal mit je 20 ml Wasser gewaschen und unter Vakuum (20 mbar) bei 50 °C getrocknet.

### Ausbeute an 4,5-Dihydro-2-(2,4-dihydroxyphenyl)-4-methylthiazol-4-(S)-carbonsäure:

6,25 g (98,5 % d. Th., DC zeigte keine signifikanten Verunreinigungen)

## Patentansprüche

1. Verfahren zur Herstellung von substituierten Thiazolinen der Formel (I) 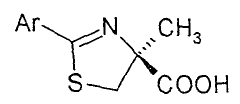 in der Ar einen Phenyl-, Naphthyl-, Thienyl-, Pyridyl- oder Chinolinylrest bedeuten, der gegebenenfalls durch ein oder mehrere Substituenten aus der Gruppe Halogen, OH, Benzyloxy, C₁-C₄-Alkyl, C₁-C₄-Alkoxy, COOR₁ mit R₁ gleich H oder C₁-C₄-Alkyl substituiert sein kann,
durch Kopplung von (S)-α-Methylcystein-Hydrochlorid der Formel (II) 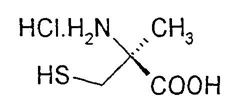 mit einem aromatischen oder heteroaromatischen Nitril der Formel (III)
Ar-CN
in der Ar wie oben definiert ist, oder einem korrespondierenden C₁-C₄-Alkylimidat, **dadurch gekennzeichnet, dass** (S)-α-Methylcystein-Hydrochlorid der Formel (II) in einem C₁-C₄-Alkohol, einem Gemisch aus zwei oder mehreren C₁-C₄-Alkoholen oder einem Gemisch aus C₁-C₄-Alkohol und einem Kohlenwasserstoff mit 0,5 bis 2 mol an einem Nitril der Formel (III) oder einem korrespondierenden C₁-C₄-Alkylimidat pro mol (S)-α-Methylcystein-Hydrochlorid in Gegenwart von 1,5 bis 3 mol einer tertiären Base pro mol (S)-α-Methylcystein-Hydrochlorid bei einem pH-Wert von 6.5 bis 10 bei 50°C bis zur Rückflusstemperatur zu dem entsprechenden Thiazolin der Formel (I) umgesetzt wird, das durch Entfernen des Lösungsmittels, Extraktion der Verunreinigungen und Ausfällen des Thiazolins durch Ansäuern aus dem Reaktionsgemisch isoliert wird.

2. Verfahren nach Anspruch 1, **dadurch gekennzeichnet, dass** als Lösungsmittel Ethanol und oder Butanol eingesetzt wird.

3. Verfahren nach Anspruch 1, **dadurch gekennzeichnet, dass** 0,8 bis 1,5 mol Nitril der Formel (III) oder C₁-C₄-Alkylimidat pro mol (S)-α-Methylcystein-Hydrochlorid eingesetzt werden.

4. Verfahren nach Anspruch 1, **dadurch gekennzeichnet, dass** Triethylamin, Di-i-Propyl-Ethylamin oder Pyridin als tert. Base in einer Menge von 1,8 bis 2,5 mol pro mol (S)-α-Methylcystein-Hydrochlorid zugesetzt werden.

5. Verfahren nach Anspruch 1, **dadurch gekennzeichnet, dass** durch die Zugabe der tert. Base in der Reaktionslösung ein pH-Wert von 7 bis 9 eingestellt wird.

6. Verfahren nach Anspruch 1, **dadurch gekennzeichnet, dass** substituierte Thiazoline der Formel (I) in der Ar einen Phenyl- oder Naphthylrest bedeutet; der gegebenenfalls durch ein oder zwei Substituenten aus der Gruppe OH, Methyl, Methoxy oder COOH substituiert sein kann, hergestellt werden.

7. Verfahren zur Herstellung des gemäß Anspruch 1 verwendeten (S)-α-Methylcystein-Hydrochlorids der Formel (II), **dadurch gekennzeichnet, dass**
a) eine L-Cystein-Verbindungen der Formel (IV) 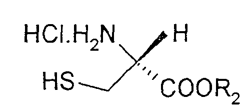 in der R₂ H oder C₁-C₄-Alkyl bedeutet, mit gegebenenfalls substituierten Nitrilen der Formel (V)
Ar-CN
in der Ar einen Phenyl-, Naphthyl-, Thienyl-, Pyridyl- oder Chinolinylrest bedeutet, der gegebenenfalls durch ein oder mehrere Substituenten aus der Gruppe Halogen, OH, Benzyloxy, C₁-C₄-Alkyl, C₁-C₄-Alkoxy, COOR₁ mit R₁ gleich H oder C₁-C₄-Alkyl substituiert sein kann, wobei die OH-Gruppen gegebenenfalls durch eine geeignete Schutzgruppe geschützt sein können, oder einem korrespondierenden C₁-C₄-Alkylimidat, in Anwesenheit von 0,9 bis 1,3 mol einer tert. Base pro mol L-Cysteinverbindung in einem C₁-C₄-Alkohol, einem Gemisch aus zwei oder mehreren C₁-C₄-Alkoholen oder einem Gemisch aus C₁-C₄-Alkohol und einem Kohlenwasserstoff zu einer Verbindung der Formel (VI) 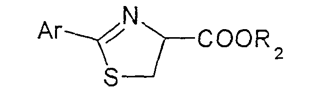 in der Ar und R₂ wie oben definiert sind,
gekoppelt wird, die
b) falls R₂ gleich H ist, in einen C₁-C₄-Ester überführt wird, dann
c) in einem Ether als Lösungsmittel mit 1 bis 3 mol eines Methylierungsreagens pro mol der Verbindung der Formel (VI) und in Anwesenheit von 1 bis 1,5 mol einer Base pro mol der Verbindung der Formel (VI) bei einer Temperatur von -80°C bis +40°C zu zum entsprechenden Thiazolin der Formel (VII) 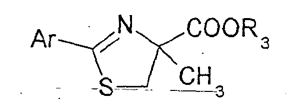 in der Ar wie oben definiert ist und R₃ C₁-C₄-Alkyl bedeutet,
methyliert wird, worauf
d) durch basische Hydrolyse die korrespondierende Thiazolincarbonsäure der Formel (VIII) 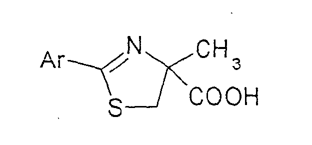 in der Ar wie oben definiert ist, als Racemat erhalten wird, wobei durch die Zugabe der Base ein zweiphasiges Reaktionsgemisch erhalten wird, das bei Rückflusstemperatur gerührt und danach abgekühlt wird, worauf die wässrige Phase mit HCI versetzt wird bis ein pH-Wert zwischen 1,5 und 4,5 erreicht ist, wodurch sich das Carbonsäure-Racemat als Öl abscheidet, das
e) durch Zugabe eines chiralen Amins als Spaltbase gespalten wird, das entsprechende Salz der Carbonsäure auskristallisiert wird und die entsprechende chirale Säure in einem mit Wasser nicht mischbaren, gegen HCI resistenten Lösungsmittel durch Zugabe von HCl bis zum Erreichen eines pH-Wertes der wässrigen Phase zwischen 1 und 4, Abtrennen der organischen Phase und des Lösungsmittels freigesetzt wird oder
f) anstelle der Racematsplatung mittels Spaltbasen eine lipasenkatalysierte Racematspaltung der Verbindung der Formel (VII) zur freien Säure durchgeführt wird, worauf
g) im Anschluss an Schritt e) oder f) die erhaltene (S)-Thiazolincarbonsäure der Formel (VIII) in HCI gelöst, bei Rückflusstemperatur gekocht und auf 0 bis 30°C abgekühlt wird, ausgefallenen Spaltprodukte abgetrennt werden, das saure Filtrat gegebenenfalls zur vollständigen Entfernung der Spaltprodukte extrahiert wird, worauf die wässrige Phase entweder sprühgetrocknet wird oder zur Gänze oder teilweise eingedampft wird und, falls nicht zur Gänze eingedampft wird, das Wasser durch Zugabe eines geeigneten Lösungsmittels durch azeotrope Destillation abgezogen wird, worauf (S)-2-Methylcystein HCl in Lösung oder als Suspension erhalten wird oder der verbleibenden Suspension oder Lösung gegebenenfalls zum Ausfällen von kristallinem ,(S)-2-Methylcystein HCl ein Ether oder ein Kohlenwasserstoff zugesetzt wird, worauf ein kristalliner Niederschlag von (S)-2-Methylcystein HCl erhalten wird.

8. Verfahren nach Anspruch 7, **dadurch gekennzeichnet, dass** als Lipase Pig Liver Esterase, Candida rugosa Lipase, Aspergillus species Lipase, Aspergillus species Protease, Bacillus species Protease, Subtilisin Carlsberg, Candida antarctica "A" Lipase, Candida antarctica "B" Lipase, ChiroCLEC_{TM-PC} (dry) oder ChiroCLEC_{TM-PC} (slurry) eingesetzt wird.

9. Verfahren zur Herstellung des gemäß Anspruch 1 verwendeten (S)-α-Methylcystein-Hydrochlorids der Formel (II), **dadurch gekennzeichnet, dass**
a) L-Cysteinethyiester HCl mit 2 bis 3 Äquivalenten Aceton in einem Kohlenwasserstoff als Lösungsmittel oder in Aceton selbst, gegebenenfalls in Anwesenheit eines tert. Amines zu dem korrespondierenden Acetonid umhesetzt wird, das
b) in 2 bis 3 Äquivalenten Ameisensäure gelöst und mit Toluol oder Essigsäureanhydrid versetzt wird, worauf das Reaktionsgemisch eingeengt und zwischen einem Ether, Ester oder geeignetem Kohlenwasserstoff und einer Natriumhydrogencarbonat-, NaOH- oder KOH-Lösung verteilt wird, die organische Phase azeotrop getrocknet und vom Lösungsmittel befreit wird, worauf
c) das so erhaltene N-formylierte Acetonid, 3-Formyl-2,2-dimethyl-thiazolin-4-carbonsäureethylester, durch Deprotonierung mit 1 bis 1,5 Äquivalenten Lithiumdiisopropylamid und anschließender Umsetzung mit 1 bis 2 Äquivalenten an Methylierungsmittel in den racemischen 3-Formyl-2,2,4-trimethyl-thiazolin-4-carbonsäureethylester überführt wird, worauf
d) mittels selektiver Esterhydrolyse unter Verwendung einer Lipase 3-Formyl-2,2,3-trimethyl-thiazolin-4-carbonsäure erhalten wird, worauf
e) eine saure Hydrolyse mittels HCl durchgeführt wird, wobei bei Rückflusstemperatur gekocht und auf 0 bis 30°C abgekühlt wird, die wässrige Phase entweder sprühgetrocknet wird oder zur Gänze oder teilweise eingedampft wird und, falls nicht zur Gänze eingedampft wird, das Wasser durch Zugabe eines geeigneten Lösungsmittels durch azeotrope Destillation abgezogen wird, worauf (S)-2-Methylcystein HCl in Lösung oder als Suspension erhalten wird oder der verbleibenden Suspension oder Lösung gegebenenfalls zum Ausfällen von kristallinem (S)-2-Methylcystein HCl ein Ether oder ein Kohlenwasserstoff zugesetzt wird, worauf ein kristalliner Niederschlag von (S)-2-Methylcystein HCl erhalten wird.

10. Verwendung von (S)-2-Methylcystein HCl, hergestellt nach Anspruch 7 oder 9, zur Herstellung von substituierten Thiazolinen der Formel (I) gemäß Anspruch 1.

11. Verfahren nach Anspruch 1, **dadurch gekennzeichnet; dass** (S)-2-Methylcystein HCl hergestellt nach Anspruch 7 oder 9 direkt nach der azeotropen Destillation im Schritt g) bzw. e) als Lösung oder Suspension, gegebenenfalls nach Ergänzung mit frischem Lösungsmittel, für die Kopplung eingesetzt wird.
